# EUROPÄISCHE PATENTSCHRIFT

(11) **EP 2 759 290 B1**
(45) Veröffentlichungstag und Bekanntmachung des Hinweises auf die Patenterteilung: **06.11.2019**
(21) Anmeldenummer: 14151146.9
(22) Anmeldetag: 14.01.2014
(51) Int. Cl.: A61K 9/00, A61K 47/10, A61K 47/34

(54) **SPRÜHBARE FLÜSSIGE ZUBEREITUNG ZUR NASALEN ANWENDUNG MIT ERHÖHTER LOKALER VERWEILZEIT**
SPRAYABLE LIQUID COMPOSITION FOR NASAL APPLICATION HAVING AN INCREASED LOCAL RETENTION TIME
COMPOSITION LIQUIDE PULVERISABLE POUR APPLICATION NASALE AVEC UN DELAI LOCAL RETENTISSANT AUGMENTE

(30) Priorität: 24.01.2013 DE 102013001151; 24.01.2013 DE 202013000748 U
(43) Veröffentlichungstag der Anmeldung: 30.07.2014
(73) Patentinhaber: Merz Pharma GmbH & Co. KGaA, 60318 Frankfurt am Main (DE)
(72) Erfinder: Berlekamp, Dr., Uwe, 48432 Rheine (DE); Möhle, Melanie, 63477 Maintal (DE)
(74) Vertreter: Müller, Claudia

(56) Entgegenhaltungen:
- WO-A2-2008/073779

## Beschreibung

### Gegenstand der Erfindung

Die vorliegende Erfindung betrifft eine tropfbare und auch sprühbare, insbesondere pharmazeutische Zubereitung zur topischen nasalen Anwendung auf Schleimhäuten auf Basis von Natriumchlorid-Salz in wässriger Lösung. Diese Zubereitung ist flüssig und kann aus entsprechenden Applikationsvorrichtungen leicht durch Tropfen oder Sprühen auf die betroffenen Schleimhäute aufgebracht werden. Dort erfolgt aufgrund der besonderen Trägergrundlage aus Wasser, einem oder mehreren Poloxamer-Tensiden und einem oder mehreren 2- und/oder 3-wertigen Alkoholen eine rasche Gelbildung. Dadurch verbleibt die Zubereitung am Wirkort ohne auszulaufen und bedingt eine sofortige Wirksamkeit. Dies ist insbesondere deshalb überraschend, weil bisher bekannte Nasalsprays auf Basis von Salz als Wirkstoff nicht zur in situ- Gelbildung geeignet waren oder für viskose Produkte besondere Gelbildner wie Acrylatpolymere, Arabisches Gummi, Dextrine u.ä. erforderlich waren, um in der Ausgangssituation bereits vor der Anwendung weniger flüssige Mittel zu erhalten.

Mit der erfindungsgemäßen Grundlagen-Wirkstoff-Kombination kann überraschender Weise ein sprühbares Produkt bereitgestellt werden, welches sofort und aufgrund der längeren Verweilzeit besonders wirksam ist. Es kann sowohl isotonisch, hypertonisch als auch hypotonisch vorliegen und wirkt rasch abschwellend aufgrund des erzielten lokalspezifischen osmotischen Effekts, ohne dass hierfür weitere, für die Nasalindikation bekannte Wirkstoffe wie Sympathomimetika, Antihistaminika oder Glukocorticosteroide vorhanden sein müssen. Das Produkt beruht daher im wesentlichen auf natürlicher Basis und ist besonders reizarm. Das oder die eingesetzten Poloxamere (Polyoxyethylen-Polyoxypropylen-Block-Polymere) weisen dabei vorzugsweise eine mittlere molare Masse von 9000 bis 15000 g/mol auf. Als Alkohole werden erfindungsgemäss Propylenglykol, Ethylenglykol, Glycerin oder Mischungen hiervon eingesetzt. Erfindungsgemäß beträgt das Gewichtsverhältnis von Poloxamer(en) zu Alkohol(en) 1,3 : 1 bis 1:1 und das Verhältnis von Wasser zur Summe aus Poloxamer(en) + Alkohol(en) 3,2 : 1 bis 3,1 : 1.

Die erfindungsgemäße Zubereitung ist geeignet bei/zur Anwendung bei Reizungen/Schwellungen der Nasenschleimhaut durch äußere Einflüsse wie Kälte, Staub, Trockenheit oder Allergene wie bei Reizungen, die durch potenziell pathogene Mikroorganismen wie Schnupfenviren (Rhinoviren, andere Erkältungsviren), bei Sinusitis, Rhinitis, oder auch bei Reizzuständen, die durch die auf die Infektion folgende Entzündung verursacht werden. Sie kann daher zur Behandlung und/oder Pflege der Nasenschleimhaut, insbesondere als Medizinprodukt, eingesetzt werden.

### Stand der Technik

Zur topischen Behandlung von entzündlichen oder irritativen Prozessen des Nasenraumes werden insbesondere Wirkstoffe oder Kombinationen eingesetzt, welche zum Abschwellen der Schleimhaut führen. Dazu gehören z. B. Xylometazolin, oder Ondensanhydrochlorid (siehe EP 1250925) oder auch Cromoglicinsäure, Azelastin, Beclometason. Derartige Wirkstoffe führen zwar zu schnellem Abschwellen der Schleimhaut, jedoch können diese bei häufiger Anwendung austrocknen, was zu erneuter Reizung, 'Nasenlaufen' und einem nicht erwünschten Gewöhnungseffekt führt.

Weniger Wirkstoffnebenwirkungen zeigt Natriumchlorid in Form von z. B. Steinsalz, oder Meersalz unterschiedlicher Provenienz. So beschreibt die DE 20 2004 012 980 U1 ein Nasenspray mit einer 0,9%igen Himalaya-Salz-Sole-Lösung, welches neben 97% Natriumchlorid noch 84 Elemente des Periodensystems enthält und besonders rein sein soll.

Die DE 100 00 612 A1 betrifft flüssige Nasal- oder Augentropfen, worin entsprechende Wirkstoffe wie Cromoglicinsäure, Xylometazolinhydrochlorid sowie 1-5 Gew.% Natriumchlorid als Konservierungsmittel neben Wasser als Träger eingesetzt werden. Dadurch sollen die durch andere Konservierungsmittel verursachten Reizungen der Schleimhaut vermieden werden.

Die WO 0100218 A1 betrifft Nasalsprays zur Behandlung von Sinusitis, Rhinitis, Epistaxis, welche Totes Meer Salz aufweisen, das 31-35% Magnesiumhalogenid, 24-26% Kaliumhalogenid, 4-8% Natriumhalogenid und 0,4 bis 0,6% Calciumhalogenid umfasst.

Die EP 0366888 A1 offenbart pharmazeutische Zubereitungen zur Behandlung entzündlicher Nasenschleimhäute, welche D- Panthenol und Kochsalz als alleinige Wirkstoffe in physiologischer Konzentration auf Basis von Wasser, ggf. mit Verdickungsmitteln, aufweisen.

Diese Zubereitungen verfügen zwar über einen reizstoffarmen, natürlichen Wirkstoff, sind jedoch flüssig bei der Anwendung, so dass nicht gewährleistet ist, dass der Wirkstoff tatsächlich in ausreichendem Maße am Wirkort verweilen kann. Sofern sie als verdickte Produkte vorliegen, ist die Wirkstoff-Anwendungsmenge begrenzt.

Daher wurden Darreichungsformen, entwickelt, welche zur einer verzögerten Freisetzung führen. So beschreibt die WO 2011050206 A2 die Verwendung von nasal anwendbaren, Gel-bildenden Zubereitungen unter Einsatz von Poloxameren wie Pluronic® F 127. Damit soll eine verzögerte Wirkstofffreisetzung von mindestens 3 Tagen vorliegen. Um diese zu erreichen, wird entweder die Menge an Poloxamer erhöht auf mehr als 15 Gew.% und/oder die Wirkstoffe werden durch geeignete Derivatisierung schwerlöslich gemacht. Weiterhin werden die Gelbildungstemperatur modifizierende Zusätze eingesetzt wie Cyclodextrin, Cellulosepolymere, PEG, Chitosane, Polyacrylate. Als nasal verabreichbare Wirkstoffe werden Immunmodulatoren, Antibiotika, Fungizide, Hormone, Calcium-Kanal-Blocker, Immunsuppressoren wie Betametason, Dexametasone, Beclometason, u.a. sowie insbesondere Corticosteroide genannt. Natriumchlorid oder analoge Salze sind als Wirkstoff nicht erwähnt.

Die EP 1051155 B (DE 69901936 T2) betrifft flüssige nasale Zubereitungen, welche als Wirkstoff Vasokonstriktoren, Antiallergika oder Corticosteroide aufweisen, und weiterhin Wasser oder Wasser im Gemisch mit Propylenglykol und/oder Glycerin mit mindestens 95% Wasser, sowie Sorbit und ein Nieder(C₁-C₄)-Alkylcellulosederivat enthalten. Damit sollen die sonst mit diesen Wirkstoffen auftretenden Nebenwirkungen wie Brennen, Stechen und vor allem das Austrocknen der Schleimhaut vermieden werden.

Die WO 94/05330 A betrifft Zubereitungen zur nasalen Verabreichung von Wirkstoffen mit Polyacrylaten sowie anionischen Tensiden.

Das Spanische Patent ES 2095183 beschreibt gelierende Zubereitungen zur intranasalen Anwendung, welche entsprechende Wirkstoffe wie Oxymetazolinhydrochlorid sowie eine Mischung aus Poloxameren mit einer mittleren Molekülmasse von 8000 D bis 12000 D mit Hydroxypropylmethylcellulose, Hydroxypropylcellulose, Acrylatcopolymeren, oder Carboxypolymethylen (Carbomer) und Natriumchlorid, jedoch keinen aliphatischen Alkohol, in ausreichenden Mengen zur Herstellung einer isotonischen Lösung enthalten.

Das Europäische Patent EP 2214658 B1 beschreibt pharmazeutische Zubereitungen, welche ebenfalls zur Vermeidung der vorstehend genannten Nebenwirkungen bzw. auch ohne Sympathomimetika mit vasokonstriktorischen bzw. schleimhautabschwellenden Eigenschaften das Austrocknen der Schleimhaut mindern können. Dazu werden Osmolyte, ausgewählt aus (Homo)-/Ectoin, Hydroxyectoin, Firoin (β-Mannosylglycer-at/amid), DGP (1,1-Di-Glyce-rinphosphat), DMIP (Di-Mannosyl-Di-Inositolphosphat), DIP (Di-myo-Inositolphosphat), c-DPG (zyclisches 2,3-Diphosphoreoglycerat), Glycosylserin eingesetzt. Die Zubereitungen sind entweder flüssig oder gelförmig (durch Hilfsstoffe wie Polyacrylate oder Celluloseether) oder können auch als Cremes oder Salbe vorliegen. Hohe Salzkonzentrationen sollen hier vermieden werden.

In der EP 1 308 169 B1 werden topisch applizierbare, bei der Anwendung gelbildende Mittel offenbart, welche einen oder mehrere in Wasser/Wasser-Alkohol schwerlösliche Wirkstoffe wie Melatonin sowie Wasser oder Wasser Alkohol-Gemische zusammen mit höheren Mengen an Poloxameren mit einer mittleren molaren Masse von 1000-18000 g/mol, insbesondere 2000-15000 g/mol aufweisen. Mit diesen flüssigen, bei der Anwendung auf der Haut, insbesondere Kopfhaut, kristalline Gel-Phasen bildenden Zubereitungen soll ein Depoteffekt und daher eine retardierte, nicht sofortige Freisetzung des Wirkstoffs erzielt werden. Poloxamer ist insofern als thermosensitiver/reversibler Gelbildner bekannt, wird aber überwiegend im Zusammenhang mit schwerwasserlöslichen Wirkstoffen eingesetzt, um auf diese Weise eine verzögerte Wirkstofffreisetzung durch Depotbildung zu erreichen.

In der WO2008/073779 A2 wird eine bioadhäsive pharmazeutische Zubereitung zur Behandlung von Angst- und Krampfzuständen beschrieben, wobei die dazu bisher beschriebenen Wirkstoffnebenwirkungen vermieden werden sollen. Diese Wirkstoffe sind Entspannungsmittel (seizuring agents) wie Barbiturate, Hydantoine, Benzodiazepine u.a. Es handelt sich dabei um nicht wasserlösliche Wirkstoffe.

### Aufgabe der Erfindung

Aufgabe vorliegender Erfindung war es, ein Mittel auf Wasserbasis bereitzustellen, dessen Hauptwirkstoff bei Anwendung im Nasalraum möglichst geringe Nebenwirkungen (wie Austrocknen der Schleimhaut) zeigt, und welches trotz seiner hohen Wirkstofflöslichkeit im Lösungsmittel auf Wasserbasis eine erhöhte Wirkortpräsenz und damit eine höhere, insbesondere sofortige Wirkeffizienz ausbildet. Insbesondere soll die Zubereitung flüssig sein, bei der Anwendung jedoch am Wirkort verbleiben.

### Lösung der Aufgabe

Diese Aufgabe wird gemäß Anspruch 1 durch eine Tropf- und sprühbare, bei Raumtemperatur (20°C) flüssige, in der Anwendung bei 35° bis 38°C gelierende Zubereitung zur topischen nasalen Anwendung wie nachfolgend beschrieben gelöst mit einem im Wesentlichen wasserlöslichen Natriumchlorid-haltigen Salz als Wirkstoff in einer Gesamtgewichtsmenge von 0,5 bis 4 Gew.%, insbesondere bis 3,5 Gew.%, in Kombination mit einer Trägergrundlage in einer Gesamtgewichtsmenge von 90 bis 96 Gew.%, insbesondere bis 96,5 Gew.% (neben 0 bis 5 Gew.%, vor allem 0,1 bis 5 Gew.% Zusatzstoffen wie nachfolgend beschrieben. Die Trägergrundlage selbst besteht hauptsächlich aus Wasser (z. B. 60 bis 88 %, bezogen auf die Trägergrundlage) und weiterhin (z. B. 40 bis 12 %, bezogen auf die Trägergrundlage) aus einer Mischung aus einem oder mehreren Poloxamer-Tensiden wie nachfolgend beschrieben mit einem oder mehreren 2-oder 3-wertigen Alkoholen, ausgewählt aus Ethylenglykol, Propylenglykol, Glycerin oder Mischungen hiervon. Das Verhältnis von Poloxamer(en) zu Alkohol(en), bezogen auf das Gesamtgewicht der Zubereitung, beträgt 1,3 zu 1 bis 1 zu 1.

Die erfindungsgemäße Nasalzubereitung besteht demnach aus:
0,5 bis 4 Gew.%, insbesondere bis 3,8 Gew.% wasserlösliches Natriumchlorid-haltiges (basiertes) Salz, ausgewählt aus Kochsalz, Steinsalz, Meersalz unterschiedlicher Provenienz;
56,5 bis 84,5 Gew.% Wasser;
6 bis 20 Gew.% eines oder mehrerer Alkohole, ausgewählt aus Ethylenglykol, Propylenglykol, Glycerin;
6 bis 20 Gew.% eines oder mehrerer Poloxamere mit einer mittleren molaren Masse von 1800 bis 18000 g/mol;
0 bis 5 Gew.% Zusatzstoffe, ausgewählt aus Zusatzwirkstoffen, wie ätherischen Ölen, wasserlöslichen Vitaminen, Proteinen, Enzymen, weiterhin pH-Wertregulatoren, Konservierungsmitteln, Konsistenzmitteln und Mischungen hiervon;
wobei das oder die Poloxamere und der oder die Alkohole in einem Gewichtsverhältnis von 1,3 : 1 bis 1:1 vorliegen.

Die Zubereitungen, vor allem pharmazeutische Mittel gemäß vorliegender Anmeldung sind geeignet zum topischen Einsatz auf Schleimhäuten des Menschen im Nasenraum. Dabei ist das anmeldungsgemäße Mittel/die Zubereitung vor allem im Wesentlichen frei von weiteren (insbesondere schwer in Wasser löslichen) Wirkstoffen, insbesondere Hauptwirkstoffen, wie vor allem anti-infektiven/steroidalen/schmerzlindernden/antiallergischen Wirkstoffen wie insbesondere Glucocorticoiden, Antihistaminika, α- Sympatomimetika. Vorzugsweise ist das Mittel weiterhin im Wesentlichen Lipid- (vor allem Triglycerid)-frei und weist einen pH-Wert von mehr als 4,5, insbesondere von mehr als 5, vor allem von 5,5 bis 8 (10%ige wässrige Lösung, RT) auf.

Gemäß vorliegender Erfindung bedeutet "pharmazeutisches Mittel" jede Art von pharmazeutischer Zubereitung, vor allem Medizinprodukte, aber auch Arzneimittel, sofern ausdrücklich jeweils benannt. Die Zubereitungen können auch als kosmetische Mittel vorliegen.

### Nähere Erläuterung der Erfindung

Die anmeldungsgemäßen Zubereitungen umfassen als Haupt-Wirkstoff Natriumchlorid in Form von Steinsalz, Meersalz, Speisesalz unterschiedlichster Provenienz. Besonders bevorzugt ist Meersalz, Totes Meer Salz, Solesalz, Speisesalz wie nachfolgend beschrieben. Dieser Wirkstoff ist weitestgehend wasserlöslich. Die erfindungsgemäße Zubereitung liegt daher vor der Anwendung (bei Raumtemperatur) als Lösung vor, da der Wirkstoff wasserlöslich und der Träger wasserhaltig ist.

Durch die Trägergrundlage aus Wasser/Alkohol und Poloxamer im angegebenen Gewichtsverhältnis geliert die Zubereitung bei der Anwendung auf der Schleimhaut entsprechend der erhöhten Temperatur (Körpertemperatur ca. 35° bis 38 °C), auch ohne die Anwesenheit weiterer bekannter mucoadhäsiver gelbildender Polymere wie Polyacrylatpolymere oder - copolymere, PEG, Arabisches Gummi, Dextrine wie Cyclodextrin, Maltodextrin, Polydextrose, Guargalactomannan, Gelatine, Chitosan, Chitosanhydrochlorid, Polyethylen-/polypropylenglycol (PEG/PPG), Siliciumdioxid, Magnesiumstearat oder Xanthan. Die erfindungsgemäße Zubereitung ist daher insbesondere im wesentlichen frei von derartig wie vorstehend genannten anderen mucoadhäsiven gelbildenden Polymeren (Acrylat(co)polymeren, Xanthan, Gummi arabicum, Chitosan, Chitosanhydrochlorid, Dextrinen, PEG/PPG oder Mischungen hiervon). Darüber hinaus sind auch keine Zuckeralkohole wie insbesondere Sorbit, Xyllit erforderlich. Die erfindungsgemäße Zubereitung weist daher vorzugsweise keine Zuckeralkohole auf und ist insbesondere frei von Sorbit, Xyllit.

Die Zubereitungen können in geeigneten Applikationsvorrichtungen wie Sprühapplikatoren ohne Treibgas bereitgesellt werden. Neben der einfachen Handhabung ist auch eine bessere Produktausnutzung aufgrund der tropfenfreien Anwendung und des dadurch vermiedenen Produktverlustes möglich. Die Erfindung betrifft insofern auch ein Nasalspray, welches zusammengesetzt ist aus einer Zubereitung wie oben beschrieben, insbesondere in der oder den bevorzugten Ausführungsformen, und welches bei Anwendung am Wirkort in situ-Gel-bildend mit Auslaufschutz ist.

Da auf Wirkstoffe mit Nebenwirkungen, insbesondere nasal applizierte Wirkstoffe mit Nebenwirkungen verzichtet werden kann, sind die erfindungsgemäßen Zubereitungen besonders verträglich.

Sie eignen sich zur topisch- mucosalen protektiven und/oder unterstützenden/oder auch kurativ-therapeutischen Anwendung auf Schleimhäuten im Nasenraum, vor allem bei Reizzuständen, ggf. Entzündungszuständen wie Sinusitis, Rhinitis, allergische Rhinitis, Schnupfen, bei Schwellungen, Trockenheit, allergischen Überreaktionen der Nasenschleimhaut. Die Zubereitungen werden vor allem auch zu nicht therapeutischen Zwecken, oder als Medizinprodukte eingesetzt. Sofern erforderlich, können geeignete Zusatzstoffe inkorporiert werden. Hierzu gehören Zusatzwirkstoffe wie Vitamin B5 (Dex(D)-panthenol, Panthenol), Aloe Vera, ätherische Öle (welche gleichzeitig auch als Riechstoffe wirken) wie vor allem Pfefferminzöl, Menthol, Eukalyptusöl, ggf. Salze wie Zinkglukonat, oder auch ggf. Enzyme, Proteine, sowie galenische Hilfsstoffe, ausgewählt aus pH-Wert-Regulatoren, Konservierungsmitteln, Konsistenzmitteln oder Mischungen hiervon.

Überraschenderweise können Reizzustände wie oben erläutert behandelt werden, wenn als Wirkstoff Natriumchlorid-Salz in einer bei Raumtemperatur flüssigen Zubereitung, enthaltend ein Wasser-Poloxamer-Alkohol-Gemisch in den genannten Mengenverhältnissen, eingesetzt wird. Dieses geliert bei der Anwendung und der Wirkstoff kann sofort, jedoch gleichzeitig auch anhaltend freigesetzt werden, ohne dass hierzu eine Depotbildung erforderlich wäre.

Dies ist deshalb überraschend, weil bisher eine ausreichende Wirkstoffverfügbarkeit nur dann mit thermoreversiblen Zubereitungen erreicht wurde, wenn der Wirkstoff verzögert, also nicht sofort freigesetzt wurde. Ohne an eine Theorie gebunden zu sein, wird angenommen, dass mit den erfindungsgemäßen Zubereitungen im beschriebenen 3-Komponentengemisch Wasser-Alkohol-Poloxamer das Wirkstoffsalz ausreichend viskos gebunden wird, so dass ein rasches Austropfen verhindert werden kann. Gleichzeitig kann eine wirkungsrelevante Salzmenge aus der flüssigen Phase freigesetzt werden. Dies konnte auch deshalb nicht erwartet werden, weil Natriumchlorid selbst als Verdickungsmittel agieren und somit seiner Funktion als Wirkstoff hier entzogen werden kann.

Nachfolgend werden die Inhaltsstoffe der anmeldungsgemäßen Zubereitungen näher beschrieben. Die Mengenangaben beziehen sich auf Gew.%, bezogen auf die Gesamtzubereitung, soweit nicht anders angegeben.

### 1. Wirkstoff

Wesentlicher (Haupt-) Wirkstoff in anmeldungsgemäßen Zubereitungen ist Natriumchlorid in Form von Mineralsalz, mit einem Hauptanteil (mindestens 70 Gew.%, vorzugsweise mindestens 75 Gew. %, oder auch mindestens 90 Gew.%, jeweils bezogen auf die Salzmischung) Natriumchlorid, insbesondere in Form von Speisesalz, Steinsalz, Meersalz, wobei je nach Herkunft die Gesamtzusammensetzung variieren kann.

In erfindungsgemäßen Zubereitungen ist daher Natriumchlorid-haltiges Mineralsalz (welches einen Hauptanteil, also mindestens 70 Gew.% Natriumchlorid aufweist) Haupt-Wirkstoff.

Mineralsalze sind Salze aus den Kationen Natrium, Kalium, Magnesium, Calcium, Eisen, Ammonium oder Mangan und den Anionen Hydrogencarbonat, Chlorid, Fluorid, Sulfat oder Nitrat, die erfindungsgemäß eingesetzt werden können, wenn wenigstens 70 Gew.%, vor allem wenigstens 90 Gew.% des Mineralsalzes aus Natriumchlorid bestehen, Dementsprechend können als Rest andere, z. B. aus dem Thermalbadmilieu und Meerwasser bekannte Ionensalze, wie z.B. solche aus den Kationen Lithium, Aluminium, Strontium, Calcium, Magnesium, und den Anionen Iodid, Bromid, Thiosulfat, Sulfat, Chlorid vorhanden sein. Deren Anteil variiert je nach Herkunft des Salzes und je nach Reinheitsgrad. Im Allgemeinen wird gereinigtes Salz eingesetzt.

Eine beispielhafte Zusammensetzung eines Meersalzes wie Biomaris umfasst: 75% Natriumchlorid, 5,6% Kaliumchlorid, 15,1% Magnesiumchlorid (Hydratisiert), 0,25 % Calciumsulfat (hydrat), 0,13% Kaliumbromid und als Rest Hydrogencarbonat, weitere lonensalze oder Mischungen hiervon.

Rohes Meersalz enthält 80 Mineralstoffe, insbesondere Natriumchlorid und bis 5% Kaliumsulfat, Magnesium-/Calciumchlorid, Mangan, Zink, Kupfer, falls es nicht gereinigt ist. Speise(Koch)salz ist entmineralisiertes Steinsalz und umfasst fast vollständig Natriumchlorid.

Der Anteil an Mineralien und Spurenelementen beim reinen Steinsalz beträgt etwa 2,5 %, bei den verbreiteten herkömmlichen, d.h. raffinierten Meersalzen liegt er bei ca. 0,8 Prozent. Naturbelassenes Meersalz (z.B. aus Tavira, Portugal) kann bis zu 4,8% Mineralien und Spurenelemente aufweisen.

Bekannte Steinsalze sind z. B.: Alpensalz, Ursalz, Quellsalz (z.B. aus Portugal), Australisches Murray River Salz, Himalaya Salz, Inka Salz, Kalahari (Ur)-Salz (z.B. Silver Crystal Gourmet Salz), Kala Namak (Indisches Schwarzsalz), Karpatensalz, Bolivianisches Salarsalz (z.B. Sel Miroir), Atlassalz, Tibet Salz, Persiensalz u. a..

Naturbelassenes Steinsalz kann folgende Zusammensetzung aufweisen: Natriumchlorid (NaCl) 97,5% min., Eisen (Fe) 5,0 - 70 mg/kg Calcium (Ca) 0,45% max., Kobalt (Co) 0,05 - 0,5 mg/kg, Magnesium (Mg) 0,05% max., Jodid (J) 0,1 - 2,2 mg/kg, Sulfat (SO₄) 1,00% max. , Zink (Zn) 0,5 - 3 mg/kg, Mangan (Mn) 0,1 - 1 mg/kg, Kalium (K) 3 - 500 mg/kg. (siehe www.j-lorber.de/gesund/salz/salzarten.htm).

Aus Steinsalz gewonnenes Speisesalz hat folgende typische Zusammensetzung:
Chlorid 59,90 %, Natrium 38,85 %, Calcium 0,25 %, Phosphor 0,15 %, Magnesium 0,12 %, Schwefel 0,02 %, Kalium <0,01 %, entsprechend 98,75% Natriumchlorid.

Die verschiedenen Salze und deren Herstellung sind bekannt. Erfindungsgemäß wird vor allem Kochsalz, Steinsalz, z.B. NaCl Ph.Eur./Südsalz eingesetzt.

Die Menge an Natriumchlorid-haltigem Mineralsalz, insbesondere Speisesalz oder Meersalz, beträgt 0,5 bis 4 Gew.%, bevorzugt 0,8 bis 3,5 Gew.%, insbesondere 1 bis 2 Gew.%, bezogen auf das Gewicht der Gesamtzusammensetzung aus (Mineral)Salz, Trägergrundlage und ggf. Zusatzstoffen. Je nach Gesamtanteil Natriumchlorid im eingesetzten Mineralsalz liegen daher ca. 0,35 bis 3,99 Gew.% Natriumchlorid (rein) vor. Es können daher hypotone, isotone oder hypertone Lösungen hergestellt werden. Insbesondere bevorzugt sind hypertone Lösungen (bezogen auf die Gesamttonizität der Lösungen).

Die erfindungsgemäßen Zubereitungen können (bezogen auf den Gehalt an Natriumchlorid) vor allem hypertonisch (1 bis 3,5 Gew.% Salz, entsprechend ca.1 bis 3 Gew.% Natriumchlorid) vorliegen. Alternativ können auch hypo-isotonische Lösungen (z.B. 0,5 bis 0,85 % Natriumchlorid) oder isotonische Lösungen (0,9 % Natriumchlorid) bereitgestellt werden.

Dementsprechend kann die Osmolarität der Zubereitung z.B. 200 bis 2500 mosmol/kg (< 270 mosmol/kg = hypoton, 270 bis ca. 310 mosmol/kg=isoton, > ca. 310 mosmol/kg= hyperton), bevorzugt > 1000 mosmol/kg, insbesondere 1200 bis 2400 mosmol/kg, vor allem 1600 bis 2350 mosmol/kg betragen. Es können also beispielsweise NaCl- isotone, (gesamt)-isotone oder NaCl- hypotone, (gesamt)hypertone Zubereitungen oder NaCl-hypertone, (gesamt)hypertone Zubereitungen vorliegen. Vor allem geeignet sind NaCl-isotone, (gesamt)-hypertone oder NaCl-hypertone, oder (gesamt)hypertone Zubereitungen.

Der pH-Wert liegt vorzugsweise bei 5,0 bis 8,5.

### 2. Poloxamer-Tensid

Poloxamere (INCI-Name) sind (Polyethylenglykol-Polypropylen)-Block-Copolymer-Tenside, insbesondere Poly(ethylene glycol)-(Kette a₁)-poly(propylene glycol)-(Kette b)-poly(ethylene glycol)(Kette a₂) -Blockpolymere der allgemeinen Formel I 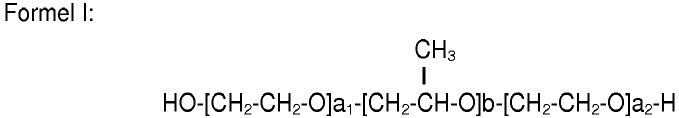 wobei der Wert a_{1,2} für die Kettenlänge a_{1,2} = 12 bis 141 (vorzugsweise 80 - 120), jeweils und der Wert b für die Kettenlänge b = 20 bis 70 (vorzugsweise 27 - 67, insbesondere 30 bis 67) beträgt.

Der HLB-Wert beträgt hier insbesondere 8-22, insbesondere 12-22. Insbesondere bevorzugt sind solche Verbindungen der Formel I, worin a₁=a₂ ist. Erfindungsgemäss sind solche Produkte mit einer relativen Molmasse von 1800 bis 18000 g/mol, insbesondere 2200-18000 g/mol, vor allem von 6000-18000 g/mol, insbesondere von 8900 bis 18000 g/mol und vor allem 9500 bis 17500 g/mol. Dabei beträgt das Verhältnis von Polyoxyethylen(POE-)a-Einheiten (∑a) zu Polyoxypropylen(POP-)-b-Einheiten bevorzugt 95:5 bis 30:70, insbesondere 85:15 bis 40:60. Insbesondere gehören zu dieser Gruppe erfindungsgemäße Poloxamere, die unter dem Handelsnamen Pluronic® bekannt sind wie Pluronic® F 127 (HLB:22), Pluronic® PE 6400, (HLB:15), Pluronic® P 65 (HLB:17); Pluronic® P123; (HLB-Wert 8). Weiterhin besonders geeignet sind Pluronic® L44NF (46:56); Pluronic® F 87 NF (72:28); Pluronic ® F 68NF (81:19); Pluronic® F 108 NF (82:18) (in Klammern ist das Verhältnis POE(Gesamtanteil a) zu POP-Anteil b) angegeben). Eine dem Produkt Pluronic® F 127 analoge Substanz ist unter dem Handelsnamen Lutrol ® F 127 (Poloxamer 407, mit ca. 73% POE , a₁₊₂= 98, b= ca. 67) bekannt.

Besonders geeignete Poloxamere sind Poloxamer 407 (auch unter dem Namen Kolliphor P 407 bekannt), molare Masse: 9840 bis 14600 g/mol; Poloxamer 338 (Lutrol F 338) (auch unter dem Namen Kolliphor P 338 bekannt), molare Masse: 12700 bis 17400 g/mol; Poloxamer 237, molare Masse: 6840 bis 8830 g/mol; Poloxamer 188, molare Masse: 7680 bis 9510 g/mol. Andere geeignete Poloxamere sind: Poloxamer 181, molare Masse: 1800 g/ mol; : Poloxamer 124, molare Masse: 2090 bis 2360 g/mol.

Das oder die Poloxamere liegen erfindungsgemäss in Mengen von 6-20 Gew. % (bezogen auf den Anteil an der Gesamtrezeptur) vor.

### 3. Alkohole

Zu den anmeldungsgemäßen Alkoholen zählen Ethylenglykol, Propylenglykol, Glycerol. Insbesondere bevorzugt ist Ethylenglykol, oder vor allem Propylenglykol oder deren Mischungen oder Mischungen aus einem oder beiden hiervon mit Glycerol wie Mischungen aus Ethylenglykol + Glycerol, Propylenglykol + Glycerol, Ethylenglykol + Glycerol + Propylenglykol. Diese Alkohole können in unterschiedlichen, jeweils geeigneten Verhältnissen gemischt werden.

Der oder die Alkohole liegen erfindungsgemäss in Mengen von 6-20 Gew. % bezogen auf die Gesamtzusammensetzung vor.

### 4.Zusatzstoffe

Die anmeldungsgemäßen Zubereitungen können weiterhin Zusatzstoffe aufweisen wie 0 bis 5 Gew.%, vor allem 0,01 bis 5 Gew.%, insbesondere 0,1 bis 4 Gew.%. Diese werden vorzugsweise ausgewählt aus ätherischen Ölen, Zusatzwirkstoffen wie Vitamine, ggf. Zinkverbindungen, Proteine oder Mischungen hiervon, sowie pH-Wertregulatoren (Säuren, Basen, Puffer), Konsistenzmitteln, Konservierungsmitteln oder Kombinationen hiervon.

### 4a) Ätherische Öle

Hierzu gehören vor allem ätherische Öle, die auch als Aroma- bzw. als Riechstoffe verwendbar sind. Dabei handelt es sich um im Allgemeinen flüchtige Extrakte aus Pflanzen, Pflanzenteilen mit dem dafür charakteristischen Geruch, die durch Zerkleinern oder vor allem durch Wasserdampfdestillation aus Pflanzen oder Pflanzenteilen gewonnen werden können.

Beispielhaft können hier genannt werden: ätherische Öle bzw. Riechstoffe aus Salbei, Thymian, Teebaum, Pfefferminze, Minzöl, (Menthol, Cineol), Eukalyptusöl, Lavendelöl, Kamillenblüten, Kiefernnadel, Zypresse, Orangen, Birkenblätter, Zimt, Limetten, Grapefruit, Mandarine, Wacholder, Baldrian, Zitronenmelisse, Zitronengras, Cranberry, Efeublätterextrakt, u. ä. oder Mischungen hiervon wie Mischungen aus Menthol, Pfefferminzöl oder Minzöl.

Die ätherischen Öle oder Mischungen hiervon können in Mengen von 0,0001 bis 1 Gew.-% (insbesondere als Mischung), vor allem 0,001 bis 0,5 Gew.-%, bevorzugt 0,01 bis 0,08 Gew.-%, bezogen auf das Gewicht der Gesamtzubereitung vorliegen. Anwendungsbedingt oder einzelfallabhängig kann es von Vorteil sein, hiervon abweichende Mengen einzusetzen. Ätherische Öle umfassen vor allem Terpene, aromatische Verbindungen, Mischungen hiervon, und gelten im Sinne vorliegender Erfindung nicht als Lipide (wie Fette/Öle).

### 4b) Zusatzwirkstoffe

Des Weiteren können auch zusätzliche Wirkstoffe wie wasserlösliche Vitamine oder Zusatzsalze oder Pflegestoffe eingesetzt werden. Hierzu gehören z. B. Vitamin C bzw. geeignete Derivate hiervon wie - Palmitat, Calcium-Ascorbat/Natrium- Ascorbat, Kalium- Ascorbat, ferner die Vitamine der B- Gruppe wie B1 (Thiamin, z. B. Thiaminnitrat), B2 (Riboflavin), B3 (Niacin, Nicotinsäure und Derivate hiervon wie Niacinamid), B5 (Pantothensäure) wie Calciumpantothenat, D(ex)-Panthenol oder Provitamin B5; B6 (Pyridoxin oder Pyridoxin-Hydrochlorid- oder -Phosphat), B9 (Folsäure), B12 (Cobalamin) oder auch Carotinoide wie Beta / Alpha-Carotin, Beta-Cryptoxanthin, Lutein, Zeaxanthin, Lycopin bzw. Aloe Vera. Besonders bevorzugt werden Vitamin C, insbesondere -palmitat oder Ca-Ascorbat, und/ oder Vitamin B5 (D(ex)-Panthenol) verwendet.

Weitere geeignete Zusatzwirkstoffe sind im Wesentlichen wasserlösliche Salze, ausgewählt aus Zinksalzen wie Zinkacetat, Zinkglukonat, oder Zinkhistidin, oder wasserlösliche Proteine.

Osmolyte wie die eingangs, nämlich im EP Patent Nr. 2214658 B1, Anspruch 1 genannten Osmolyte sind erfindungsgemäß nicht erforderlich. Die Zubereitungen sind daher vorzugsweise frei von derartigen Osmolyten.

Die einzelnen Zusatzwirkstoffe bzw. Derivate hiervon werden in den dafür geeigneten Mengen z.B. zwischen 0,001 und 2% jeweils, insgesamt z. B. in einer Menge von 0,01 bis 3 Gew. %, bezogen auf die Gesamtgewichtsmenge der Zubereitung, eingesetzt. Andere Zusatzwirkstoffe, insbesondere solche, die nicht in Wasser- oder Wasser/Alkohol- Gemischen löslichen Wirkstoffe, wie sie im Stand der Technik für die Nasalapplikation genannt sind wie insbesondere Corticosteroide, Antihistaminika, Vasokonstriktoren, oder Alkycellulosederivate, müssen anmeldungsgemäß nicht eingesetzt werden. Es kann also auf diese Substanzen verzichtet werden. Ebenso kann auf weitere Tenside wie z.B. solche mit einem HLB-Wert von 8 und mehr oder auch W/O-Tenside (wie Wollwachsalkohole) und lipophile Substanzen wie Phospholipide, Fettsäuren und deren Salze, Glyceride verzichtet werden. Die erfindungsgemäßen Zubereitungen sind daher bevorzugt im wesentlichen frei von diesen genannten Substanzen.

Vorzugsweise besteht die erfindungsgemäße Zubereitung daher aus:
0,5 bis 4 Gew.% Mineralsalz mit mindestens 70 Gew.% vorzugsweise mit mindestens 90 Gew. %, bezogen auf die Salzmischung, Natriumchlorid-Salz als Hauptwirkstoff, ausgewählt aus Speisesalz, Steinsalz, Meersalz unterschiedlicher Provenienz;
56,5 bis 84,5 Gew.% Wasser; 6 bis 20 Gew.%, insbesondere 10-13 Gew.%, vor allem > 10 bis 13 Gew.%, eines oder mehrerer Alkohole, ausgewählt aus Ethylenglykol, Propylenglykol, Glycerin, oder Mischungen hiervon; 6 bis 20 Gew-%, insbesondere 10 bis 13 Gew.%, vor allem > 10 bis 13 Gew.%, eines oder mehrerer Poloxamer-Tenside (Polyoxyethylen-Polyoxypropylen-Blockpolymere) mit einer mittleren molaren Masse von 9000 bis 18000 g/mol; 0 bis 5 Gew.%, vor allem 0,01 bis 5 Gew.% Zusatzstoffe, ausgewählt aus Zusatzwirkstoffen der Gruppe aus ätherischen Ölen, wasserlöslichen Vitaminen, ggf. Zinksalzen, Proteinen oder Mischungen hiervon, weiterhin pH-Wertregulatoren, Konservierungsmitteln, Konsistenzmitteln und Mischungen hiervon, wobei das Gewichtsverhältnis des/der Poloxamere zu dem oder den Alkoholen 1,3 : 1 bis 1:1 beträgt.

Die Osmolarität der Gesamtzubereitung beträgt vorzugsweise 1300 bis 2400 mosmol/kg.

### 4c) pH-Wert- Regulatoren

Zu den geeigneten pH-Wert-Regulatoren zählen vor allem Puffer wie Phosphat-, Carbonat-Sulfat- oder Zitratpuffer (z. B. Natrium-/Kalium-(hydrogen)-phosphat/carbonat), sowie Säuren wie Zitronensäure, Weinsäure, Sorbinsäure, vor allem Kaliumcitrat, Natriumcitrat, oder Basen wie Natriumcarbonat, Natriumhydrogenphosphat.

Diese Substanzen liegen in jeweils im Zusammenhang mit der Gesamtzubereitung vorliegenden geeigneten Mengen vor, insbesondere derart, dass der pH-Wert der erfindungsgemäßen Zubereitung vorzugsweise zwischen 5,5 und 8 liegt.

### 4d) Konservierungsmittel

Konservierungsmittel sind im Allgemeinen in der erfindungsgemäßen Zubereitung nicht erforderlich, können aber ggf. eingesetzt werden. Hierzu eignen sich vor allem: Sorbate, Bezoate wie Benzoesäure, Anissäure. Diese Substanzen sind dem Fachmann bekannt.

### 4e) Konsistenzmittel

Gegebenenfalls können Konsistenzmittel (Konsistenzmodulatoren) wie Celluloseether, z.B. Hydroxyethylcellulose, Hydroxypropylcellulose, Methylcellulose, Hydroxypropylmethylcellulose, oder Komplexbildner wie EDTA, in geringen Mengen von 0,01 - 2,0 % eingesetzt werden. Andere Konsistenzmittel, insbesondere Lipide, W/O- und/ oder andere O/W-Tenside sind bevorzugt nicht enthalten.

### Bevorzugte Ausführungsformen

Bevorzugte Ausführungsformen umfassen insbesondere Zubereitungen, die bestehen aus:
0,8 bis 3,5 Gew.%, vor allem bis 2 Gew. % Mineralsalz mit mindestens 70 Gew.%, vor allem auch mit mindestens 90 Gew.%, insbesondere mit mindestens 95 Gew.%, bezogen auf die Salzmischung, Natriumchlorid-Salz, als Hauptwirkstoff, ausgewählt aus Speisesalz, Steinsalz, Meersalz unterschiedlicher Provenienz;
71-82 % Wasser;
9 bis 13 Gew.% eines oder mehrerer Alkohole, ausgewählt aus Ethylenglykol, Propylenglykol, Glycerin oder Mischungen hiervon;
9 bis 13 Gew. % eines oder mehrerer Poloxamer-Tenside (Polyoxyethylen-Polyoxypropylen-Blockpolymere) mit einer mittleren molaren Masse von 1800 bis 10000 oder 6000 bis 18000, oder auch vor allem von 9500 bis 17500 g/mol;
0,01 bis 3 Gew. % Zusatzstoffe, ausgewählt aus ätherischen Ölen, wasserlöslichen Vitaminen, pH-Wertregulatoren, Konservierungsmitteln, und Mischungen hiervon, wobei das oder die Poloxamere und der oder die Alkohole und in einem Gewichtsverhältnis von 1, 3 : 1 bis 1,2:1 vorliegen.

Die Mengen an einzelnen Inhaltsstoffen sind jeweils so gewählt, dass eine 100%-Mischung vorliegt.

Als Alkohol wird vor allem Propylenglykol gewählt.

Bevorzugt ist Propylenglykol bzw. das Poloxamer-Tensid, insbesondere Lutrol® F 127, in Mengen von jeweils 10 Gew.% bzw. 13 Gew.% vorhanden.

Als Poloxamer wird insbesondere Lutrol® F 127 (= Kolliphor P 407) (Poloxamer 407, molare Masse: 9840 bis 14600 g/mol) eingesetzt.

Auch Lutrol F338/ Kolliphor P338 ist geeignet.

Wasser (vor allem gereinigtes Wasser) ist bevorzugt in Mengen von 75 bis 79 Gew.% vorhanden.

Als Salz, insbesondere in Mengen von 1 bis 3,5 Gew.% Salz (bezogen auf die Gesamtzusammensetzung), entsprechend 0,9 bis 3 Gew.% Natriumchlorid, wird vor allem Steinsalz, Meersalz, raffiniertes Steinsalz, raffiniertes Meersalz oder Mischungen hiervon eingesetzt. Die Viskosität erfindungsgemäßer flüssiger Zubereitungen beträgt bei Raumtemperatur (20 °C) vor allem 50 - 100 mPas, bei der Anwendung bei Körpertemperatur steigt sie auf Werte um 150 mPas ± 30.

Der pH-Wert erfindungsgemäßer Zubereitungen liegt vorzugsweise bei 6 bis 8,5.

### Herstellung

Erfindungsgemäße flüssige Zubereitungen sind im wesentlichen klar und werden bevorzugt bei Raumtemperatur durch Mischen des Salzes in (sterilem) Wasser unter Zugabe des Poloxamers in Alkohol/Wasser und ggf. von Zusatzstoffen/ggf. Zusatzwirkstoffen hergestellt. Gegebenenfalls kann eine Sterilfiltration vorgenommen werden.

Die Zubereitungen werden sodann in die jeweils gewünschten Behälter gefüllt wie z.B. Sprühapplikatoren, Sprayflaschen (Pump- und Ventilsprays) mit beispielsweise 10 bis 100 ml (vorzugsweise 20 ml), z. B. mit dosiertem Austrag, aus Glas oder Kunststoff mit einer jeweils geeigneten Austragvorrichtung, vorzugsweise ohne Treibgas. Die Zubereitungen können daraus durch Sprühen oder Tropfen verabreicht werden und sind lagerstabil.

### Anwendung

Die anmeldungsgemäßen Zubereitungen werden eingesetzt zur topisch-nasalen unterstützenden, protektiven/ und/oder kurativen Anwendung auf der Nasenschleimhaut, insbesondere bei Menschen, bei Irritationen, Schwellungen und Reizungen, die durch Infektionen, (pathogene Mikroorganismen), daraus entstandene Entzündungen, durch Trockenheit, Kälte oder andere Reize, Allergene entstanden sind. Die Zubereitungen eignen sich auch zur Pflege der Nasenschleimhaut und sind gut verträglich. Durch die vorliegende Menge an Natriumchlorid erfolgt eine Sofortwirkung aufgrund des bekannten osmotischen Effekts, da der Wirkstoff am Wirkort wegen der erfolgten Viskositätsänderung in hoher Konzentration vorliegt.

Insbesondere werden die anmeldungsgemäßen topischen Anwendungsformen, wie vor allem pharmazeutische Zubereitungen (zu denen Medizinprodukte und Arzneimittel (therapeutisch) oder nicht therapeutische Produkte gehören) in Form von treibgasfreien versprühbaren Lösungen, Sprays, Tropfen eingesetzt. Die anfangs flüssigen Zubereitungen werden bei der Anwendung auf die Schleimhaut viskoser bis gelartig, so dass sie nicht auslaufen. Die beschriebenen Zubereitungen sind daher sowohl tropf-und sprühbar als auch gleichzeitig auslaufgeschützt. Der frei gesetzte Wirkstoff Natriumchlorid (Salz) verbleibt am Ort und entfaltet seine oben beschriebene Wirkung sofort.

### Beispiele

Die Erfindung wird anhand der nachfolgenden Beispiele, die den beschriebenen Gegenstand jedoch nicht beschränken, näher erläutert.

**Beispiel 1 Tropfbares hypertones Nasenspray mit Steinsalz/Polypropylenglykol**

| Inhaltsstoff | Chemische Bezeichnung / Funktion | 100% Rezeptur |
|---|---|---|
| Wasser | Aqua ph.Eur. | 72,779 |
| Lutrol F127/ Kolliphor P407 | Polyethylen-polypropylen- Glykol | 13 |
| Popylenglykol | 1,2-Propandiol | 10 |
| Glycerin 86% | Glycerol | |
| Natriumchlorid | Sodium Chloride | 3 |
| Meersalz (Biomaris) | Meersalz | |
| Steinsalz | Speisesalz | |
| Dexpanthenol | D-Panthenol | 1 |
| Pfefferminzöl | Mentha piperita oil | 0,01 |
| Eukalyptusöl | Eucalyptus globulus leaf oil | 0,03 |
| Kaliumhydrogenphosphat | Potassium hydrogen phosphate | 0,056 |
| Kaliumdihydrogenphosphat | Potassium dihydrogen phosphate | |
| Dermosoft 688 | Para Anissäure | 0,125 |
| | | 100 |

**Beispiel 2 NaCl-Isotone Nasallösung**

| Inhaltsstoff | Chemische Bezeichnung / Funktion | 100% Rezeptur |
|---|---|---|
| Wasser | Aqua ph.Eur. | 74,879 |
| Lutrol F127/ Kolliphor P407 | Polyethylen-polypropylen- Glykol | 13 |
| Popylenglykol | 1,2-Propandiol | |
| Glycerin 86% | Glycerol | 10 |
| Natriumchlorid | Sodium Chloride | |
| Meersalz (Biomaris) | Meersalz | 0,9 |
| Steinsalz | Speisesalz | |
| Dexpanthenol | D-Panthenol | 1 |
| Pfefferminzöl | Mentha piperita oil | 0,01 |
| Eukalyptusöl | Eucalyptus globulus leaf oil | 0,03 |
| Kaliumhydrogenphosphat | Potassium hydrogen phosphate | 0,056 |
| Kaliumdihydrogenphosphat | Potassium dihydrogen phosphate | 0,125 |
| Dermosoft 688 | Para Anissäure | |
| | | 100 |

**Beispiel 3 Hypertone Nasallösung**

| Inhaltsstoff | Chemische Bezeichnung / Funktion | 100% Rezeptur |
|---|---|---|
| Wasser | Aqua ph.Eur. | 74,279 |
| Lutrol F127 /Kolliphor P407 | Polyethylen-polypropylen- Glykol | 13 |
| Popylenglykol | 1,2-Propandiol | 10 |
| Glycerin 86% | Glycerol | |
| Natriumchlorid | Sodium Chloride | |
| Meersalz (Biomaris) | Meersalz | |
| Steinsalz | Speisesalz | 1,5 |
| Dexpanthenol | D-Panthenol | 1 |
| Pfefferminzöl | Mentha piperita oil | 0,01 |
| Eukalyptusöl | Eucalyptus globulus leaf oil | 0,03 |
| Kaliumhydrogenphosphat | Potassium hydrogen phosphate | 0,056 |
| Kaliumdihydrogenphosphat | Potassium dihydrogen phosphate | 0,125 |
| Dermosoft 688 | Para Anissäure | |
| | | 100 |

**Beispiel 4 Hypertone Nasallösung**

| Inhaltsstoff | Chemische Bezeichnung / Funktion | 100% Rezeptur |
|---|---|---|
| Wasser | Aqua ph.Eur. | 72,779 |
| Lutrol F338/ Kolliphor P338 | Polyethylen-polypropylen- Glykol | 13 |
| Popylenglykol | 1,2-Propandiol | 10 |
| Glycerin 86% | Glycerol | |
| Natriumchlorid | Sodium Chloride | 3 |
| Meersalz (Biomaris) | Meersalz | |
| | | |
| Steinsalz | Speisesalz | |
| Dexpanthenol | D-Panthenol | 1 |
| Pfefferminzöl | Mentha piperita oil | 0,01 |
| Eukalyptusöl | Eucalyptus globulus leaf oil | 0,03 |
| Kaliumhydrogenphosphat | Potassium hydrogen phosphate | 0,056 |
| Kaliumdihydrogenphosphat | Potassium dihydrogen phosphate | 0,125 |
| Dermosoft 688 | Para Anissäure | 0,05 |
| | | 100 |

**Beispiel 5 Hypertones Nasalspray**

| Inhaltsstoff | Chemische Bezeichnung / Funktion | 100% Rezeptur |
|---|---|---|
| Wasser | Aqua ph.Eur. | 72,729 |
| Lutrol F127/ Kolliphor P407 | Polyethylen-polypropylen- Glykol | 13 |
| Popylenglykol | 1,2-Propandiol | |
| Glycerin 86% | Glycerol | 10 |
| Natriumchlorid | Sodium Chloride | 3 |
| Meersalz (Biomaris) | Meersalz | |
| Steinsalz | Speisesalz | |
| Dexpanthenol | D-Panthenol | 1 |
| Pfefferminzöl | Mentha piperita oil | 0,01 |
| Eukalyptusöl | Eucalyptus globulus leaf oil | 0,03 |
| Kaliumhydrogenphosphat | Potassium hydrogen phosphate | 0,056 |
| Kaliumdihydrogenphosphat | Potassium dihydrogen phosphate | 0,125 |
| Dermosoft 688 | Para Anissäure | 0,05 |
| | | 100 |

## Patentansprüche

1. Tropf- und sprühbare, bei 20°C Raumtemperatur flüssige, in der Anwendung bei 35-38° C gelierende Nasalzubereitung zur topischen nasalen Anwendung auf der Nasenschleimhaut mit Wirkort-Wirksamkeit, bestehend aus:
0,5 bis 4 Gew. % Mineralsalz mit mindestens 70 Gew. %, bezogen auf die Salzmischung, Natriumchlorid-Salz, als Hauptwirkstoff, ausgewählt aus Kochsalz, Steinsalz, Meersalz unterschiedlicher Provenienz;
56,5 bis 84,5 Gew.% Wasser;
6 bis 20 Gew.% eines oder mehrerer Alkohole, ausgewählt aus Ethylenglykol, Propylenglykol, Glycerin, oder Mischungen hiervon;
6 bis 20 Gew.% eines oder mehrerer Poloxamer-Tenside (Polyoxyethylen-Polyoxypropylen-Blockpolymere) mit einer mittleren molaren Masse von 1800 bis 18000 g/mol;
0 bis 5 Gew.%, Zusatzstoffen, ausgewählt aus ätherischen Ölen, wasserlöslichen Vitaminen, Enzymen, Proteinen und Zinksalzen, weiterhin pH-Wertregulatoren, Konservierungsmitteln, Konsistenzmitteln, oder Mischungen hiervon,
wobei das oder die Poloxamere und der oder die Alkohole in einem Gewichtsverhältnis von 1,3 : 1 bis 1:1 vorliegen.

2. Zubereitung zur topischen nasalen Anwendung nach Anspruch 1, **dadurch gekennzeichnet, dass** sie einen oder mehrere Zusatzstoffe, ausgewählt aus ätherischen Ölen, wasserlöslichen Vitaminen, pH-Wert-Regulatoren, Konservierungsmitteln oder Kombinationen hiervon aufweist.

3. Zubereitung zur topischen nasalen Anwendung nach Anspruch 1 oder 2, **dadurch gekennzeichnet, dass** sie im Wesentlichen frei ist von nicht in Wasser oder in Wasser-Alkohol- Mischungen löslichen Wirkstoffen.

4. Zubereitung zur topischen nasalen Anwendung nach einem der Ansprüche 1 bis 3, **dadurch gekennzeichnet, dass** das/die Poloxamer-Tensid(e) ausgewählt ist/sind aus Poloxameren der allgemeinen Formel I 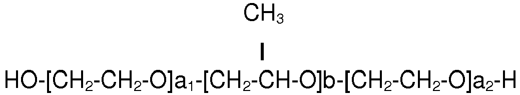
wobei der Wert für a_{1,2} jeweils zwischen 80 - 120 und für b 30 - 67 beträgt,
mit einer mittleren Molmasse von 9500 bis 17500 g/mol.

5. Zubereitung zur topischen nasalen Anwendung nach einem der Ansprüche 1 bis 4, **dadurch gekennzeichnet, dass** als Alkohol Propylenglykol ausgewählt ist.

6. Zubereitung zur topischen nasalen Anwendung nach einem der Ansprüche 1 bis 5, **dadurch gekennzeichnet, dass** als Poloxamer ein (Polyethylenglykol-Polypropylen)-Block-Copolymer mit einer mittleren molaren Masse von 9840 bis 14600 g/mol (wie die unter dem Handelsnamen Kolliphor 407 bekannte Substanz) ausgewählt ist.

7. Zubereitung zur topischen nasalen Anwendung nach einem der Ansprüche 1 bis 6, **dadurch gekennzeichnet, dass** das Verhältnis von Poloxamer-Tensid zu Alkohol 1, 3 : 1 beträgt.

8. Zubereitung zur topischen nasalen Anwendung nach einem der Ansprüche 1 bis 7, **dadurch gekennzeichnet, dass** sie 10 Gew. %, bezogen auf die Gesamtzubereitung, Alkohol, 13 Gew.%, bezogen auf die Gesamtzubereitung, Poloxamer und 1 bis 3,5 Gew.% Salz, entsprechend 0,9 bis 3 Gew.%, bezogen auf die Gesamtzubereitung, Natriumchlorid-basiertes Salz, aufweist.

9. Zubereitung zur topischen nasalen Anwendung nach einem der Ansprüche 1 bis 8, **dadurch gekennzeichnet, dass** sie hyperton ist.

10. Zubereitung zur topischen nasalen Anwendung nach einem der Ansprüche 1 bis 9 zur topischen, protektiven, pflegenden und/oder unterstützenden Anwendung bei Reizungen/Schwellungen der Nasenschleimhaut durch Trockenheit, Allergien, pathogene Mikroorganismen, Entzündungen der Schleimhäute im Nasenraum.

11. Zubereitung zur topischen nasalen Anwendung nach einem der Ansprüche 1 bis 10, **dadurch gekennzeichnet, dass** sie in Form eines Medizinproduktes zur topischen, protektiven und/oder unterstützenden Anwendung auf die Nasenschleimhaut vorliegt.

12. Zubereitung zur topischen nasalen Anwendung nach einem der Ansprüche 1 bis 11, **dadurch gekennzeichnet, dass** sie zu 10 bis 100 ml in einem Spray- oder Sprühapplikator enthalten ist.

## Claims

1. A drippable and sprayable nasal preparation, which is liquid at room temperature of 20°C and gels on administration at 35-38°C, for topical nasal administration on the nasal mucosa with efficacy at the site of action, consisting of:
0.5 to 4 wt.% of mineral salt with at least 70 wt.%, based on the salt mixture, of sodium chloride salt as the main active ingredient, selected from common salt, rock salt, sea salt of differing origin;
56.5 to 84.5 wt.% of water;
6 to 20 wt.% of one or more alcohols, selected from ethylene glycol, propylene glycol, glycerol, or mixtures thereof;
6 to 20 wt.% of one or more poloxamer surfactants (polyoxyethylenepolyoxypropylene block polymers) with an average molar mass of 1800 to 18000 g/mol;
0 to 5 wt.% of additives, selected from essential oils, water-soluble vitamins, enzymes, proteins and zinc salts, furthermore pH regulators, preservatives, consistency agents, or mixtures thereof,
wherein the poloxamer(s) and the alcohol(s) are present in a weight ratio of 1.3:1 to 1:1.

2. A preparation for topical nasal administration according to claim 1, **characterised in that** it comprises one or more additives selected from essential oils, water-soluble vitamins, pH regulators, preservatives or combinations thereof.

3. A preparation for topical nasal administration according to claim 1 or 2, **characterised in that** it is substantially free of active ingredients which are insoluble in water or in water-alcohol mixtures.

4. A preparation for topical nasal administration according to one of claims 1 to 3, **characterised in that** the poloxamer surfactant(s) is/are selected from poloxamers of the general formula I 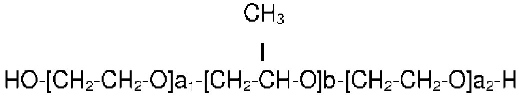
wherein the value for a_{1,2} in each case amounts to between 80 and 120 and for b to between 30 and 67,
with an average molar mass of 9500 to 17500 g/mol.

5. A preparation for topical nasal administration according to one of claims 1 to 4, **characterised in that** propylene glycol is selected as the alcohol.

6. A preparation for topical nasal administration according to one of claims 1 to 5, **characterised in that** a (polyethylene glycol-polypropylene) block copolymer with an average molar mass of 9840 to 14600 g/mol (such as the substance known by the trade name Kolliphor 407) is selected as the poloxamer.

7. A preparation for topical nasal administration according to one of claims 1 to 6, **characterised in that** the ratio of poloxamer surfactant to alcohol amounts to 1.3:1.

8. A preparation for topical nasal administration according to one of claims 1 to 7, **characterised in that** it comprises 10 wt.%, based on the total preparation, of alcohol, 13 wt.%, based on the total preparation, of poloxamer and 1 to 3.5 wt.% of salt, corresponding to 0.9 to 3 wt.%, based on the total preparation, of sodium chloride-based salt.

9. A preparation for topical nasal administration according to one of claims 1 to 8, **characterised in that** it is hypertonic.

10. A preparation for topical nasal administration according to one of claims 1 to 9 for topical, protective, caring and/or supportive administration in the event of irritation/swelling of the nasal mucosa due to dryness, allergies, pathogenic microorganisms or inflammation of the mucous membranes in the nasal cavity.

11. A preparation for topical nasal administration according to one of claims 1 to 10, **characterised in that** it takes the form of a medical product for topical, protective and/or supportive administration onto the nasal mucosa.

12. A preparation for topical nasal administration according to one of claims 1 to 11, **characterised in that** is present in a spray applicator or atomizer in an amount of 10 to 100 ml.

## Revendications

1. Préparation nasale instillable et pulvérisable pour application topique nasale sur la muqueuse nasale, liquide à 20° C à température ambiante et devenant un gel lors de l'utilisation à 35-38° C, dotée d'une activité sur le site d'action, constituée de :
0,5 à 4 % en poids de sel minéral avec au moins 70 % en poids par rapport au mélange de sels, de sel de chlorure de sodium comme principe actif principal, choisi parmi le sel de table, le sel gemme, et le sel marin de provenance différente ;
56,5 à 84,5 % en poids d'eau ;
6 à 20 % en poids d'un ou plusieurs alcools choisis parmi l'éthylène glycol, le propylène glycol, le glycérol ou un mélange de ceux-ci ;
6 à 20 % en poids d'un ou plusieurs tensioactifs poloxamères (polymères séquencés polyoxyéthylène-polyoxypropylène) ayant une masse molaire moyenne de 1800 à 18000 g/mol ;
0 à 5 % en poids d'additifs choisis parmi les huiles essentielles, les vitamines hydrosolubles, les enzymes, les protéines et les sels de zinc, de même que les régulateurs de pH, les agents de conservation, les texturants ou leurs mélanges ; le ou les poloxamères et le ou les alcools étant présents selon un rapport pondéral de 1,3:1 à 1:1.

2. Préparation pour application topique nasale selon la revendication 1, **caractérisée en ce qu'**elle comprend un ou plusieurs additifs choisis parmi les huiles essentielles, les vitamines hydrosolubles, les régulateurs de pH, les conservateurs ou des combinaisons de ceux-ci.

3. Préparation pour application topique nasale selon la revendication 1 ou 2, **caractérisée en ce qu'**elle est essentiellement exempte de substances actives insolubles dans l'eau ou dans des mélanges eau-alcool.

4. Préparation pour application topique nasale selon l'une des revendications 1 à 3, **caractérisée en ce que** le ou les tensioactifs poloxamères sont choisis parmi les poloxamères de formule générale I : 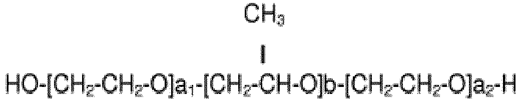 où la valeur de a_{1,2} est à chaque fois comprise entre 80 et 120 et celle de b entre 30 et 67, ayant une masse molaire moyenne de 9500 à 17500 g/mole.

5. Préparation pour application topique nasale selon l'une des revendications 1 à 4, **caractérisée en ce que** l'alcool choisi est le propylène glycol.

6. Préparation pour application topique nasale selon l'une des revendications 1 à 5, **caractérisée en ce que** le poloxamère choisi est un copolymère séquencé (propylène glycol - propylène) ayant une masse molaire moyenne de 9840 à 14600 g/mol (telle que la substance connue sous le nom commercial Kolliphor 407).

7. Préparation pour application topique nasale selon l'une quelconque des revendications 1 à 6, **caractérisée en ce que** le rapport du tensioactif poloxamère relativement à l'alcool est de 1,3:1.

8. Préparation pour application topique nasale selon l'une des revendications 1 à 7, **caractérisée en ce qu'**elle comprend 10% en poids d'alcool par rapport à la préparation totale, 13% en poids de poloxamère par rapport à la préparation totale et 1 à 3,5% en poids de sel, correspondant à 0,9 à 3% en poids de sel de chlorure de sodium par rapport à la préparation totale.

9. Préparation pour application topique nasale selon l'une des revendications 1 à 8, **caractérisée en ce qu'**elle est hypertonique.

10. Préparation pour application topique nasale selon l'une des revendications 1 à 9 pour application topique protectrice, thérapeutique et/ou de soutien en cas d'irritation/gonflement de la muqueuse nasale provoqué(e) par la sécheresse, des allergies, des microorganismes pathogènes, ou une inflammation des muqueuses de la cavité nasale.

11. Préparation pour application topique nasale selon l'une des revendications 1 à 10, **caractérisée en ce qu'**elle se présente sous la forme d'un produit médical pour application topique, protectrice et/ou de soutien sur la muqueuse nasale.

12. Préparation pour application topique nasale selon l'une des revendications 1 à 11, **caractérisée en ce qu'**elle est contenue en quantité de 10 à 100 ml dans un applicateur à nébulisation ou pulvérisation.
